# EUROPEAN PATENT APPLICATION

(11) **EP 1 402 811 A1**
(43) Date of publication of application: **31.03.2004**
(21) Application number: 02743763.1
(22) Date of filing: 28.06.2002
(51) Int. Cl.: A61B 5/00, G06F 17/60, G06T 1/00

(54) **IMAGE DELIVERY APPARATUS**

(30) Priority: 05.07.2001 JP 2001204790
(71) Applicant: KONICA CORPORATION, Tokyo 163-0512 (JP)
(72) Inventor: YAMAMICHI, Youji, Hino-shi, Tokyo 191-8511 (JP)
(74) Representative: Rees, Alexander Ellison
(86) International application number: PCT/JP2002/006567
(87) International publication number: WO 2003/003913

(57) **Abstract**

The present invention provides an image distributing apparatus, which makes it possible to automatically and appropriately classify destination terminals, to which the image information sets are distributed, without requiring the hospital to prepare extra personnel for this operation, and as a result, to improve the efficiency of the operation. The image distributing apparatus is provided with: a memory means for storing the image information, formed by image-forming apparatus, etc., such as a Computer Tomographic Scanner, a Magnetic Resonance image-forming apparatus and an X-ray image-forming apparatus (including Computed Radiography), and to which the retrieving information are added; an inquiring means for inquiring of an information managing server of a hospital information system about destination terminals of the image information based on the retrieving information; and a distributing means for distributing the image information to an image viewer equipped in a medical room, an image viewer equipped in an image inspection room, an image viewers equipped in a filing room, etc., each serving as a destination terminal inquired by the inquiring means.

## Description

### TECHNICAL FIELD OF THE INVENTION

The present invention relates to an image distributing apparatus equipped in a hospital, which distributes image information including added retrieval information, formed by CT Scanner (Computer Tomographic Scanner), Magnetic Resonance image-forming apparatus, X-ray image-forming apparatus (including Computed Radiography), etc., to imaging devices, such as image viewers, provided at doctor's sites, at each of which a doctor makes diagnosis for a patient based on the retrieval information concerned.

### TECHNICAL BACKGROUND OF THE INVENTION

Conventionally, there has been put to practical use an image recording/reproducing system, which displays medical images on the image viewer provided at a doctor's site based on the image information and store the image information in the image server equipped at a filing room, after image-forming apparatus, such as CT Scanner (Computer Tomographic Scanner), Magnetic Resonance image-forming apparatus, X-ray image-forming apparatus (including Computed Radiography), etc., captures the medical image of the patient to generate the image information and an appropriate image-processing is applied to the image information.

When distributing the image information to image viewers, etc., provided at appropriate medical examination and treatment rooms (hereinafter, referred to as medical rooms or a medical room, for simplicity) among a large number of medical rooms and image inspection rooms in the hospital, for instance, a specific personnel had to recognize the information, such as the name of the patient, the ID number, the date of photographing, the date of patient's birth, etc., by referring to descriptions in a form filled by the doctor to specify destination terminals appropriate for distributing the image information, and then, has inputted information with respect to the destination terminals into the image distributing apparatus. When it is required to change the destination terminals to new of them because of certain reasons, for instance, the personnel had to cope with such the situation by re-inputting information with respect to revised destination terminals.

Conventionally, since specific personnel has inputted information with respect to the destination terminals into the image distributing apparatus when distributing the image information to the image viewers, etc., provided at appropriate medical rooms, image inspection rooms, etc., it has been a problem that not only the hospital should employ extra personnel for this operation, but also manual inputting operations have lowered the efficiency of the whole image reading operation based on the image information. For instance, in case of error occurrences in the distribution processing when a plurality of doctors treat one patient, a plurality of image information sets, generated in a medical examination, need to be classified into plural categories, each of which correspond to each of the plurality of doctors. In the above case, if the excessive number of the image information sets are transmitted to the image viewer for a certain doctor or a certain inspection room, an enormous amount of time would be consumed for retrieving the necessary image information sets, resulting in the deterioration in efficiency for the whole image reading operation.

To overcome the abovementioned drawbacks in conventional image distributing apparatus, it is an object of the present invention to provide an image distributing apparatus, which makes it possible to automatically and appropriately classify destination terminals, to which the image information sets are distributed, without requiring the hospital to prepare extra personnel for this operation, and as a result, to improve the efficiency of the operation.

### DISCLOSURE OF THE INVENTION

The abovementioned object of the present invention can be attained by image distributing apparatus described as follow.
(1) An image distributing apparatus characterized by comprising: an image information acquiring means for acquiring image information by photographing a subject to be inspected; a memory means for adding retrieving information to the image information and for storing them in it; an inquiring means for inquiring of an information managing server of a hospital information system, which stores and controls information pertaining to the retrieving information, about destination terminals of the image information based on the retrieving information; and a distributing means for distributing the image information to the destination terminals inquired by the inquiring means.
(2) An image distributing apparatus characterized by comprising: an image information acquiring means for acquiring image information by photographing a subject to be inspected; a memory means for adding retrieving information to the image information and for storing them in it; an determining means for acquiring information pertaining to the retrieving information from an information managing server of a hospital information system, which stores and controls information pertaining to the retrieving information, to determine destination terminals of the image information; and a distributing means for distributing the image information to the destination terminals determined by the determining means.
(3) The image distributing apparatus, recited in item (1) or item (2), characterized in that the image information acquiring means is a Computer Tomographic Scanner, a Magnetic Resonance image-forming apparatus, a X-ray image-forming apparatus (including Computed Radiography), etc.
(4) The image distributing apparatus, recited in anyone of items (1)-(3), characterized in that the retrieving information includes a name of a patient, an ID number of the patient, a name of a requested doctor, etc.
(5) The image distributing apparatus, recited in anyone of items (1)-(4), characterized in that the destination terminals are image viewers equipped in medical rooms, image inspection rooms, etc. pertaining to the retrieving information among all of medical rooms, image inspection rooms, etc.
(6) The image distributing apparatus, recited in anyone of items (1)-(4), characterized in that the retrieving information are transferred to a terminal device at the image-forming apparatus side by operating a terminal device at the image viewer side, and are added to the image information.
(7) An image distributing apparatus characterized by comprising: an image information acquiring means for acquiring image information by means of a photographing apparatus, which photographs a patient, being a subject to be inspected, to form the image information; an adding means for adding patient information pertaining to the patient and photographing information pertaining to a photographing operation; an inquiring means for inquiring of an information managing system, which controls requesting information in regard to the photographing operation for the patient, about the requesting information corresponding to the patient information and the photographing information added by the adding means; an determining means for determining destination terminals of the image information by using the requesting information inquired and acquired by the inquiring means; and a distributing means for distributing the image information to the destination terminals determined by the determining means.
(8) The image distributing apparatus, recited in item (7), characterized in that, in case that the patient information and the photographing information, added by the adding means, include specific information, a memory means, for storing the image information to which patient information and the photographing information including the specific information are added, is provided.
(9) The image distributing apparatus, recited in item (7) or item (8), characterized in that the determining means determines a plurality of destination terminals by using the requesting information, and then, the distributing means distributes the image information to the plurality of destination terminals determined by the determining means.
(10) The image distributing apparatus, recited in item (9), characterized in that the determining means determines an image viewer equipped in a medical room, an image viewer equipped in an image inspection room, an image viewer equipped in a surgical operation room or an image server for controlling the image information as one of destination terminals by using the requesting information.
(11) The image distributing apparatus, recited in anyone of items (7)-(10), characterized in that the requesting information include information of a requested medical section or a requested doctor who requests a photographing operation of the patient.
(12) The image distributing apparatus, recited in item 7, characterized in that the photographing apparatus is a Computer Tomographic Scanner, a Magnetic Resonance image-forming apparatus or a X-ray image-forming apparatus (including Computed Radiography).
(13) The image distributing apparatus, recited in items 12, characterized in that the patient information and the photographing information are transferred to the image-forming apparatus from a terminal device equipped at a destination terminal, and are added to the image information.

### BRIEF DESCRIPTION OF THE DRAWINGS

Fig. 1 shows a block diagram of a configuration of image distributing apparatus 1 embodied in the present invention as a first embodiment.
Fig. 2 shows a flowchart, indicating an example of a processing operation of image distributing apparatus 1 embodied in the present invention as a first embodiment.
Fig. 3 shows a block diagram of a configuration of image distributing apparatus 2 embodied in the present invention as a second embodiment.
Fig. 4 shows an example of requesting information controlled by RIS 11 shown in Fig. 3.
Fig. 5 shows a flowchart, indicating an example of a processing operation of image distributing apparatus 2 embodied in the present invention as a second embodiment.

### BEST MODE FOR IMPLEMENTING THE INVENTION

### <FIRST EMBODIMENT>

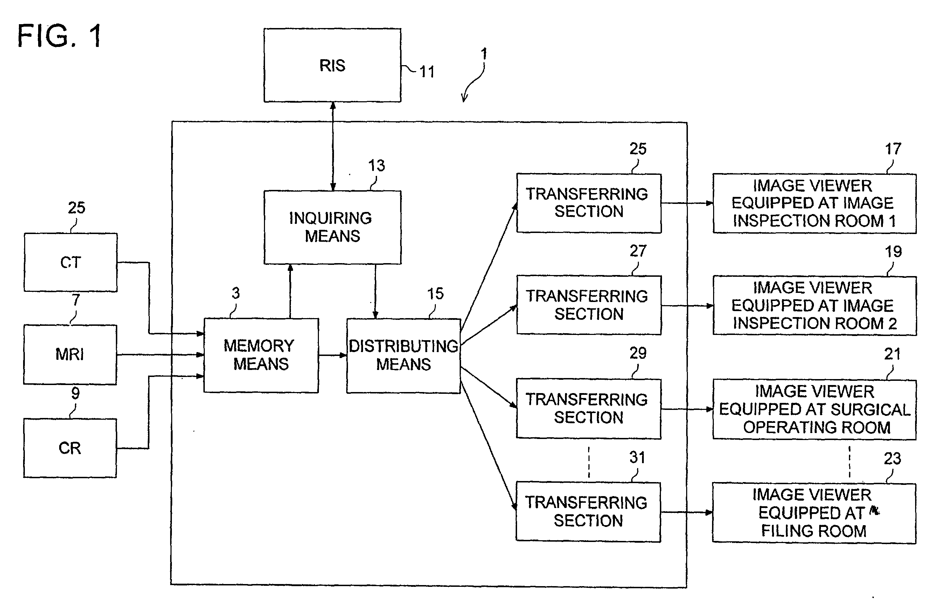

Referring to the drawings, the first embodiment of the present invention will be detailed in the following. Fig. 1 shows a block diagram for explaining the image distributing apparatus embodied in the present invention. In Fig. 1, numeral 1 indicates an image distributing apparatus provided with memory means 3, which stores image information generated by photographing an internal organ of a human body serving as a subject by means of an image-forming apparatus (an image information acquiring apparatus) and retrieval information added to the header of the image information concerned. Generally speaking, CT 5 (Computer Tomography), MRI 7 (Magnetic Resonance Image-forming apparatus), CR 9 (Computed Radiography categorized in X-ray image-forming apparatus), etc. are employed as the image-forming apparatus.

The retrieval information includes the name of the patient, the ID number, the date of patient's birth, the receipt number, the name of the doctor originally requested, the medical examination and treatment section (hereinafter, referred to as the medical section, for simplicity) originally accepted, the name of the reference doctor, the title of modality, the status of photographing mode, etc. When adding the retrieval information to the image information, there are two practical modes. In one of the two practical modes, for instance, a photographing inspection personnel operates a terminal equipped at CT 5, MRI 7 or CR 9, each serving as an image-forming apparatus, to input necessary items in it by referring to descriptions in a form filled by the doctor originally requested by the patient, while, in another one of the two practical modes, for instance, the doctor, originally requested by the patient, operates a terminal of an image viewer equipped at his own medical room to input necessary items in it and then to transmit the inputted necessary items to the terminal equipped at CT 5, MRI 7 or CR 9.

Image distributing apparatus 1 comprises inquiring means 3, which reads the retrieval information of the header when the image information, outputted from CT 5, MRI 7 or CR 9, each serving as an image-forming apparatus, are stored in memory means 3, so that the retrieval information concerned and an inquiring request for the destination terminal of the image information are outputted to RIS 11, serving as a management server of the hospital information system, and the destination terminal of the image information concerned can be recognized by receiving a reply information sent from RIS 11.

Incidentally, medical information of all patients, names of doctors in charge of medical treatments for all patients, information in regard to the medical room for each doctor, and as information in respect to materials and equipments, information about connecting terminals of image viewers equipped at each of medical rooms, information about connecting terminals of image viewers 17, 19 equipped at each of image inspection rooms 1, 2, information about a connecting terminal of image viewer 21 equipped at a surgical operating room, information about a connecting terminal of image server 23 equipped at a filing room, etc., -- etc. are stored in RIS 11. Further, RIS 11 has a function for specifying the information in regard to the destination terminal of the image information (namely, the information about connecting terminals of image viewers and/or image server) in response to the request sent from inquiring means 13 by collating the stored information mentioned above with the retrieval information, and then, returning the specified information to inquiring means 13.

Further, distributing means 15 is coupled to inquiring means 13. When distributing means 15 recognizes the destination terminal of the image information on the basis of the information returned from RIS 13, distributing means 15 outputs the information in regard to the destination terminal of the image information and a command for transferring the image information stored in memory means 3 to the destination terminal concerned.

Although details are not shown in the drawing, the distributing means 15 is coupled to a plurality of transferring sections 25, 27, 29, ---, 31, each of which is further coupled to the image viewer equipped at each of medical rooms, each of image viewers 17, 19 equipped at each of image inspection rooms 1, 2, image viewer 21 equipped at the surgical operating room, image server 23 equipped at a filing room, etc., in relation of one to one connecting mode. When distributing means 15 receives the command for transferring the information in regard to the destination terminal of the image information and the image information to the destination terminal concerned, distributing means 15 select a specific transferring section, which is coupled to the destination terminal concerned, out of the plurality of transferring sections 25, 27, 29, ---, 31, and transfers the image information stored in memory means 3 to the destination terminal selected. When activated as a result of the above selection, each of transferring sections 25, 27, 29, ---, 31 transfers the image information to the image viewer equipped at one of the medical rooms or one of image viewers 17, 19 equipped at each of image inspection rooms 1, 2, etc., which is coupled to the transferring section concerned.

Sometimes, a plurality of image information sets are acquired in the photographic inspection of a certain patient. As for such the case, there are two different cases. In one of the two cases, for instance, the retrieval information, added to the leading image information set, includes such information that destination terminals for all of the image information sets, acquired through all inspection operations for the patient, are the same, while, in another one of the two cases, for instance, destination terminals of the image information sets are different relative to each other by adding the different retrieval information, such as the name of the doctor originally requested, to each of the image information sets, so as to cope with such a case that both a doctor of internal medicine and a surgery doctor treat the same patient. All of the image information sets are simultaneously transmitted to image server 23 equipped at the filing room to store them in it, when distributing them to the image viewers equipped at the specific medical rooms, image inspection rooms, etc.

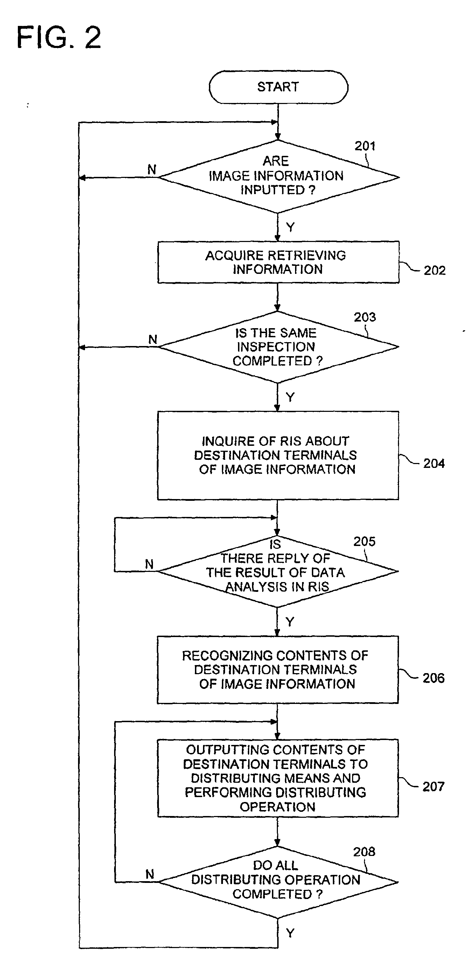

Referring to the flowchart shown in Fig. 2, an example of operating processes of the first embodiment will be detailed in the following. Initially, when determining that the image information are inputted in step 201, inquiring means 13 is activated to acquire the retrieval information from the header of the image information concerned in step 202. Then, in step 203, it is determined whether or not all inspection operations in regard to one patient are entirely completed. When not in step 203, next operating step is to return to step 201. When determined that all inspection operations in regard to one patient are entirely completed in step 203, inquiring means 13 inquires of RIS 11 about the destination terminal of each of the image information sets in step 204. With respect to the operation for inquiring about the destination terminals, it is an operating mode to transfer all of the retrieval information in regard to the image information to RIS 11 and to output a command for requesting a reply from, for instance, the medical room corresponding to the name of the doctor currently retrieving the information, or a reply of information about a connecting terminal of the image viewer equipped in the image inspection room.

Then, when a result of the data-analyzing operation performed in RIS 11 determines that the information in regard to the destination terminal of each image information exist in step 205, inquiring means 13 recognizes the contents of the destination terminal of each image information in step 206, and further in step 207, outputs them to distributing means 15, so as to allow distributing means 15 to perform distributing operation of the image information sets concerned. In case that distributing means 15 performs the distributing operation, for instance, when destination terminals of a certain image information set are established at both image viewer 17 in image inspection room 1 and image server 23 in the filing room, both shown in Fig. 1, distributing means 15 retrieves the image information concerned from memory means 3 and transfers them to transferring sections 25, 31, so as to allow transferring sections 25, 31 to perform the transferring operations. Then, the judgment, for whether or not all of the transferring operations are completed, is made in step 208. When not in step 208, the operation of step 207 is repeated, and when determining that all of the transferring operations are completed, the next is to return to step 201.

Since the first embodiment is so constituted that the retrieval information are added to all of the image information to be stored in memory means 3 to establish the destination terminal of each image information set, for instance, when a plurality of doctors make diagnosis and treat one patient, it becomes possible to automatically and easily perform such an operation that a plurality of image information sets, generated in a medical examination, are appropriately classified into plural categories, each of which correspond to a need of each of the plurality of doctors. Accordingly, it also becomes possible to eliminate such the disadvantage that the excessive number of the image information sets are transmitted to the image viewer for a certain doctor or a certain inspection room and an enormous amount of time would be forcibly consumed for selecting a necessary image information set or for retrieving the necessary image information sets.

Incidentally, it is also possible to easily structure inquiring means 13 in such a manner that, instead of recognizing the destination terminals of the image information after receiving the result of the data analyzing operation performed in RIS 11, by obtaining the information pertaining to the name of the patient, the ID of the patient, etc., from the retrieval information stored in RIS 11, inquiring means 13 itself finds out the information in regard to the destination terminals form the obtained information.

### <SECOND EMBODIMENT>

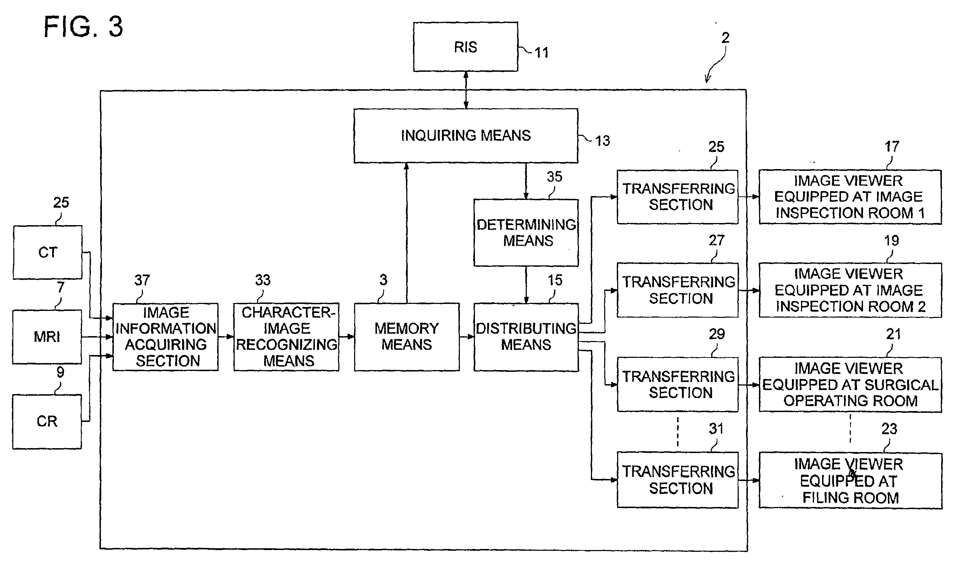

Fig. 3 shows a block diagram for explaining an image distributing apparatus, embodied in the present invention and serving as a second embodiment. As shown in Fig. 3, image distributing apparatus 2, serving as the second embodiment of the present invention, comprises memory means 3, inquiring means 13, distributing means 15, transferring sections 25 - 31, character-image recognizing means 33, determining means 35 and image information acquiring section 37. Incidentally, in Fig. 3, the same means and sections as those in image distributing apparatus 1 of the first embodiment have the same reference number as those attached to them.
Image information acquiring section 37 acquires image information generated by the image-forming apparatus, such as CT 5, MR 17, CR 9, and outputs them to character-image recognizing means 33. At this time, image information acquiring section 37 also acquires patient information and photographing information pertaining to the image information concerned, as well as the image information concerned.

The primary function of character-image recognizing means 33 is to recognize character-image information included in the overall image information and to output the recognized character-image information to memory means 3. Incidentally, although character-image recognizing means 33 is equipped in the second embodiment so as to acquire the patient information and the photographing information by recognizing the character-image information when the patient information and the photographing information are described as the character-image information included in the image information, this character-image recognizing means is not necessary required, but provided for coping with such a case, in which it is necessary to recognize the character-image information when the patient information and the photographing information are described as the character-image information included in the image information.

Memory means 3 serves as a memory means for temporally storing the image information, inputted from image-forming apparatus, such as CT 5, MR 17, CR 9, to which the patient information and the photographing information are added as header information, and outputs the stored image information to distributing means 15 when determining means 35 determines the destination terminal. In other words, memory means 3 also has a function of an adding means. Incidentally, in an emergency case that medical images of a first-aid patient are emergently photographed, specific information, for instance, patient ID of "9999", which indicates a first-aid patient, are added to the header information of the image information, instead of inputting details of his patient information and the photographing information into the header information of each image information set. In the above case, it is impossible for image server 23 to conduct the image-managing operation of the medical images of the first-aid patient when the image information sets are sent to image server 23 as they are, due to the lack of the detailed patient information and the photographing information. Accordingly, it is also applicable that memory means 3 keeps storing the image information in it without transferring them to image server 23, until inquiring means 13 obtains the detailed patient information and the photographing information by performing the inquiring operation for RIS 11.

Inquiring means 13 transmits the patient information and/or the photographing information, both described in the header information of the image information stored in memory means 3, to RIS 11, and inquires requesting information corresponding to the image information based on those information. Then, inquiring means 13 receives inquiring results from RIS 11 to output them to determining means 35.

Determining means 35 determines the destination terminal by employing the requesting information obtained as the result of the inquiring operation, and outputs the information in regard to the determined destination terminal to distributing means 15.

Distributing means 15 outputs the image information inputted from memory means 3 to one of transferring sections 25 - 31, which corresponds to the information in regard to the destination terminal inputted from determining means 35.

Further, the distributing means 15 is coupled to transferring sections 25, 27, 29, ---, 31, each of which corresponds to each of the transferring destinations, such as image viewer 17 equipped at image inspection rooms 1, image viewer 19 equipped at image inspection rooms 2, image viewer 21 equipped at a surgical operating room and image server 23 equipped at a filing room, and is coupled to each of them in relation of one to one connecting mode. Upon receiving the image information and a transferring command, each of transferring sections 25, 27, 29, ---, 31 transmits the image information to the transferring destination concerned. Incidentally, Fig. 3 is a conceptual schematic diagram, indicating that each of transferring sections 25, 27, 29, ---,

31 is a transferring means for transferring the image information by changing each of the transferring destinations. Further, the transferring destinations shown in Fig. 3 are an example of the above. For instance, the image viewer, etc. equipped at each of medical sections could be established as a transferring destination, which might not be limited to a specific category as far as being appropriate for the medical activities.

Further, image distributing apparatus 2 is coupled to image-forming apparatus including CT 5, MR 17, CR 9, etc., RIS 11 which performs the managing operation of the requesting information in regard to the medical examination of the patient by means of the image-forming apparatus, image viewers 17, 19 equipped at image inspection rooms 1, 2, image viewer 21 equipped at a surgical operating room, image server 23 which is equipped at a filing room and performs the managing operation of all of the image information sets photographed in the hospital, the image viewer equipped at each of the medical sections (not shown in the drawings), etc.

RIS 11 stores medical information of all patients, names of doctors in charge of medical treatments for all patients, information in regard to the medical section to which each doctor belongs, and as information in respect to materials and equipments of all medical sections, information about connecting terminals of image viewers equipped at each of medical rooms, information about connecting terminals of image viewers 17, 19 equipped at each of image inspection rooms 1, 2, information about a connecting terminal of image viewer 21 equipped at a surgical operating room, information about a connecting terminal of image server 23 equipped at a filing room, etc., in it. Further, RIS 11 has a function for returning an inquiring result corresponding to the inquiry from image distributing apparatus 2.

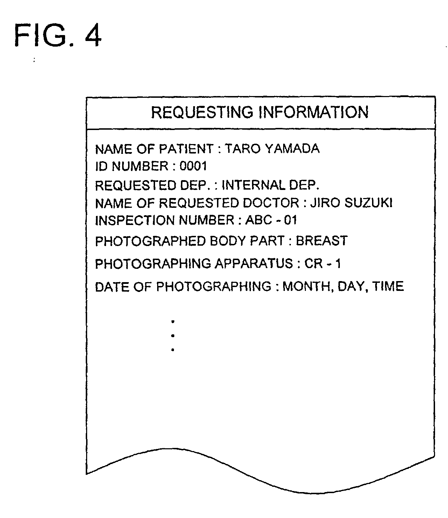

Further, RIS 11 controls requesting information in respect to inspection photographing operations for all patients. As shown in an example of Fig. 4, the requesting information includes information in regard to inspection request for every patient, such as the name of the patient, the ID number of the patient, the medical section to which the inspection photographing operation is requested, the name of the requested doctor, the inspection number, the body part to be photographed, the photographing apparatus, the date of photographing operation, etc. Incidentally, the information included in the requesting information is not limited to the abovementioned items. It may be applicable that the information also includes other items, such as the date of patient's birth, the patient's sex, photographing posture, photographing method, etc. Namely, RIS 11 is an information management system for controlling the requesting information in respect to the inspection photographing operation of the patient.

The photographing apparatus of CT 5, MR 17 and CR 9, serving as image-forming apparatus for generating image information by photographing the patient as a subject, outputs the patient information, such as the name of the patient, the ID number of the patient, etc., and the photographing information, such as the date of photographing operation, the photographing apparatus, etc., to image distributing apparatus 2, as well as the image information concerned. Incidentally, there are some operating modes for acquiring the patient information and the photographing information, which are outputted with the image information by the image-forming apparatus. For instance, in one of the above operating modes, a photographic inspection personnel operates the terminal device of the image-forming apparatus to input the necessary items by referring the predetermined inspection requesting form in which the requested doctor filed the necessary items, while, in another one of the above operating modes, the requested doctor operates the terminal device of the image viewer equipped at the medical room to input the necessary items and to transfer them to the image-forming apparatus concerned, or in still another one of the above operating modes, a receipt personnel, who has receipted the inspection request, operates the terminal device of RIS 11 to input the necessary items and to transfer them to the image-forming apparatus concerned.

Further, when generating a plurality of image information sets in respect to one patient, information, indicating that each of image information sets is generated in the same inspecting operation with respect to one patient, are included in the header information of each of the image information sets, and in addition, information, indicating that the each of the image information sets is transferred to the same destination terminal, are also included.

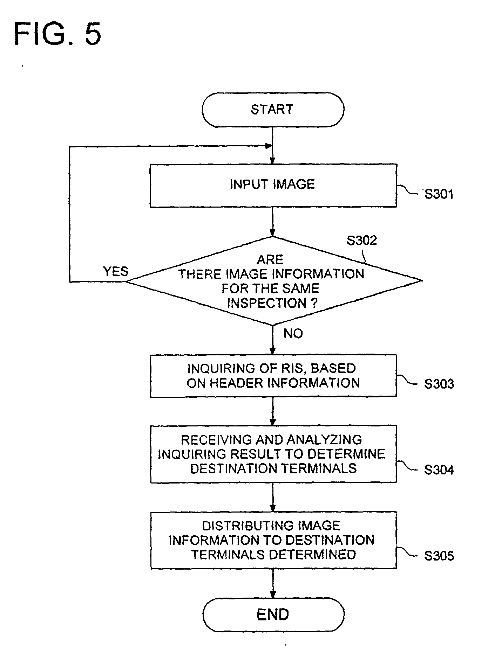

Next, referring to the flowchart shown in Fig. 5, an example of the image distributing operation in the second embodiment will be detailed in the following. Incidentally, it is desirable that the image distributing operation detailed in the following is performed at every time when the image information are inputted from the image-forming apparatus of CT 5, MR 17, CR 9, etc.

Initially, the image information are generated by the image-forming apparatus of CT 5, MR 17 or CR 9, and, when the generated image information as well as the patient information and the photographing information concerned are inputted into image distributing apparatus 2 from image information acquiring section 37 (step S301 shown in Fig. 5), the patient information and the photographing information are added to the inputted image information as the header information in order to store them in memory means 3. In this step, if the patient information and the photographing information concerned are included in the image information as character-image information, character-image recognizing means 33 will recognize the character-image information to acquire the patient information and the photographing information concerned and to add them to the image information.

The next step is to determine whether or not another image information set with respect to one patient in the same inspecting operation should be further acquired (step S302 shown in Fig. 5). When determining that another image information set in the same inspecting operation should be further acquired in step S302, the step will return to step S301, to receive reentry of the other image information and to store them in memory means 3 one by one.

On the other hand, when not in step S302, namely when all of the image information sets with respect to one patient in the same inspecting operation are already inputted, inquiring means 13 readouts the patient information or the photographing information from the header information of the image information stored in memory means 3, and inquires of RIS 11 about the requesting information corresponding to all image information included in the inspecting operation of one patient on the basis of the patient information or the photographing information concerned (step S303). Concretely speaking, for instance, if the patient information include the ID number of the patient or the photographing information include information indicating the date of photographing operation and the photographing apparatus, inquiring means 13 inquires and requests the requesting information, which coincide with each of the information. RIS 11 retrieves concerned requesting information, which coincide with the patient information or the photographing information sent from image distributing apparatus 2, from the requesting information under the management operation of RIS 11, and then, transmits them to image distributing apparatus 2.

When inquiring means 13 receives the requesting information from RIS 11 as a result of the inquiring operation, inquiring means 13 outputs the concerned requesting information to determining means 35, which analyzes the concerned requesting information to determine the destination terminal (step S304). The operation for determining the destination terminal will be detailed in the following. At first, determining means 35 determines image server 23, equipped in the filing room to control the image information, as one of destination terminals, so as to allow all of the image information to be transferred to image server 23.

Next, referring to the requesting information, for instance, when the name of the medical section concerned is filled in the item of the requesting section, determining means 35 determines the image viewer (not shown in the drawings) equipped in the medical section concerned as one of the destination terminals. Otherwise, when the name of the doctor is filed in the item of the requested doctor, determining means 35 determines the image viewer equipped in the image inspection room for the requested doctor, for instance, image viewers 17 equipped in image inspection room 1, as one of the destination terminals. Further, when the ID number of, for instance, "9999", which indicates a first-aid patient, is filled in the item of the patient ID since the image information is acquired by photographing the first-aid patient, determining means 35 determines image viewer 21 equipped in the surgical operation room, as one of the destination terminals.

Concretely speaking with reference to Fig. 4, for instance, when the internal department is filed as the requested section and the name of "Taro Yamada" is filed as the requested doctor using image inspection room 1 in the requesting information, the image viewer terminal device equipped in the internal department, image viewer 17 equipped in image inspection room 1 and image server 23 equipped in the filing room are determined as the destination terminals.

Incidentally, the abovementioned condition for determining the destination terminals is only an example of them, and therefore, the determining condition is not limited to the above.

Further, in case that there are plural doctors in charge of one patient, for instance, when both a doctor of internal medicine and a surgical doctor engage in the medical treatment of one patient, the names of the plural doctors are filed in the item of the requested doctor, and the terminal devices, each being set for each of the plural doctors, are determined as the destination terminals.

When determining means 35 determines the destination terminals in the manner mentioned above, the destination terminal information, which indicate the destination terminals, are outputted to distributing means 15, and memory means 3 outputs the image information for the same inspecting operation, serving as a transferring object, to distributing means 15. Distributing means 15 outputs the image information inputted from memory means 3 to the transferring section(s), (each) being one of transferring sections 25, 27, 29, 31, which corresponds to the destination terminal information inputted from determining means 35, and commands the transferring operation to distribute the image information (step S305).

Each of the transferring sections, which receives the transferring command, transfers the concerned image information to the corresponding destination terminal, and finalizes the image distributing operation in image distributing apparatus 2. Incidentally, concretely speaking with the abovementioned example shown in Fig. 4, when the image viewer terminal device equipped in the internal department, image viewer 17 equipped in image inspection room 1 and image server 23 equipped in the filing room are determined as the destination terminals, the image information, inputted from memory means 3 and serving as a transferring object, are outputted to the transferring section corresponding to the image viewer terminal device equipped in the internal department (not shown in the drawings), transferring section 25 corresponding to image viewer 17 equipped in image inspection room 1 and transferring section 23 corresponding to image server 31 equipped in the filing room, and then, transfer to each of the destination terminals determined.

According to the second embodiment of the present invention, since the requesting information, corresponding to the image information concerned, are obtained by inquiring RIS 11 on the basis of the patient information or the photographing information acquired from the header information of the image information concerned, and one or plural destination terminal(s), to which the image information are distributed, is/are determined by employing the requesting information concerned, it becomes possible to automatically perform such an operation as suitably categorizing the images into each of the destination terminal(s), and therefore, it becomes possible to alleviate the burden of the user who conventionally has performed such the categorizing operation. In addition, it also becomes possible to eliminate such the disadvantage that an enormous time has consumed for selecting necessary image information set(s) or retrieving each of the image information set(s), because of the fact that too many image information sets, exceeding the necessary number, were transmitted to one doctor or one image inspection room.

According to the present invention, the following effects can be obtained.
(1) Since the image distributing apparatus is structured by comprising: the memory means for storing the image information, to which the retrieving information are added, in it; the inquiring means for inquiring of the information managing server of the hospital information system about destination terminals of the image information based on the retrieving information; and the distributing means for distributing the image information to the destination terminals inquired by said inquiring means, it becomes possible to automatically and appropriately categorize the destination terminals of the image information without requiring the hospital to prepare extra personnel for this operation, resulting in an improvement of the operating efficiency.
(2) Since the image distributing apparatus is structured by comprising: the memory means for storing the image information, to which the retrieving information are added, in it; the determining means for acquiring information pertaining to the retrieving information from the information managing server of the hospital information system, in order to determine destination terminals of the image information; and the distributing means for distributing the image information to the destination terminals determined by said determining means, it becomes possible to automatically and appropriately categorize the destination terminals of the image information without requiring the hospital to prepare extra personnel for this operation, resulting in an improvement of the operating efficiency.
(3) Since the abovementioned image-forming apparatus is a Computer Tomographic Scanner, a Magnetic Resonance image-forming apparatus, an X-ray image-forming apparatus (including Computed Radiography), etc., it is effective to improve the quality of the medical treatments in the hospital.
(4) Since the retrieving information includes the name of the patient, the ID number of the patient, the name of the requested doctor, etc., it is easy to retrieve the destination terminals of the image information, resulting in an improvement of the processing efficiency.
(5) Since the destination terminals are image viewers equipped in medical rooms, image inspection rooms, etc. pertaining to said retrieving information among all of medical rooms, image inspection rooms, etc., the functionability of them is high as destination terminals of the image information, and it becomes possible to deliver high-quality images to the requested doctor.
(6) Since the retrieving information are transferred to a terminal device at the image-forming apparatus side by operating a terminal device at the image viewer side, and are added to the image information, the retrieving information are high-reliable, and convenient for improving the operating efficiency of the personnel, etc. in the hospital.
(7) Since the inquiring means inquires about the requesting information based on the patient information and the photographing information added to the image information and the determining means automatically determines destination terminals of the image information by using the requesting information inquired and acquired by the inquiring means and the distributing means distributes the image information to the destination terminals, it becomes possible to automatically and appropriately categorize the destination terminals of the image information without requiring the hospital to prepare extra personnel for this operation, resulting in an improvement of the operating efficiency.
(8) In case that the patient information and the photographing information, added to the image information, include specific information, since the image information is stored in the memory means, it is possible to temporally store the image information.
(9) Since a plurality of destination terminals are determined by using the requesting information to distribute the image information, it is possible to establish a plurality of destination terminals at a same time, resulting in an improvement of the distributing efficiency.
(10) Since the image viewer equipped in the medical room, the image viewer equipped in the image inspection room, the image viewer equipped in the surgical operation room or the image server for controlling said image information is determined as one of destination terminals by using the requesting information, it becomes possible to deliver the image information most suitable for the requested doctor and effective for medical treatment activities.
(11) Since the requesting information include information of the requested medical section or the requested doctor, it becomes easy to determine the destination terminals, resulting in an improvement of the distribution efficiency.
(12) Since the photographing apparatus is the Computer Tomographic Scanner, the Magnetic Resonance image-forming apparatus or the X-ray image-forming apparatus (including the Computed Radiography), such the photographing apparatus is effective for improving the quality of medical examinations and treatments in the hospital.
(13) Since the patient information and the photographing information are transferred to the image-forming apparatus from the terminal device equipped at the destination terminal, and are added to the image information, it becomes possible not only to maintain the patient information and the photographing information at a high reliable level, but also to improve the working efficiency of the personnel in the hospital.

## Claims

1. An image distributing apparatus **characterized by** comprising:
an image information acquiring means for acquiring image information by photographing a subject to be inspected;
a memory means for adding retrieving information to said image information and for storing them in it;
an inquiring means for inquiring of an information managing server of a hospital information system, which stores and controls information pertaining to said retrieving information, about destination terminals of said image information based on said retrieving information; and
a distributing means for distributing said image information to said destination terminals inquired by said inquiring means.

2. An image distributing apparatus **characterized by** comprising:
an image information acquiring means for acquiring image information by photographing a subject to be inspected;
a memory means for adding retrieving information to said image information and for storing them in it;
a determining means for acquiring information pertaining to said retrieving information from an information managing server of a hospital information system, which stores and controls information pertaining to said retrieving information, to determine destination terminals of said image information; and
a distributing means for distributing said image information to said destination terminals determined by said determining means.

3. The image distributing apparatus, recited in claim 1 or claim 2, **characterized in that** said image information acquiring means is a Computer Tomographic Scanner, a Magnetic Resonance image-forming apparatus, an X-ray image-forming apparatus (including Computed Radiography), etc.

4. The image distributing apparatus, recited in anyone of claims 1-3, **characterized in that** said retrieving information includes a name of a patient, an ID number of said patient, a name of a requested doctor, etc.

5. The image distributing apparatus, recited in anyone of claims 1-4, **characterized in that** said destination terminals are image viewers equipped in medical rooms, image inspection rooms, etc. pertaining to said retrieving information among all of medical rooms, image inspection rooms, etc.

6. The image distributing apparatus, recited in anyone of claims 1-4, **characterized in that** said retrieving information are transferred to a terminal device at said image-forming apparatus side by operating a terminal device at said image viewer side, and are added to said image information.

7. An image distributing apparatus **characterized by** comprising:
an image information acquiring means for acquiring image information by means of a photographing apparatus, which photographs a patient, being a subject to be inspected, to form said image information;
an adding means for adding patient information, pertaining to said patient, and photographing information, pertaining to a photographing operation, to said image information acquired by said image information acquiring means;
an inquiring means for inquiring of an information managing system, which controls requesting information in regard to said photographing operation for said patient, about said requesting information corresponding to said patient information and said photographing information added by said adding means;
a determining means for determining destination terminals of said image information by using said requesting information inquired and acquired by said inquiring means; and
a distributing means for distributing said image information to said destination terminals determined by said determining means.

8. The image distributing apparatus, recited in claim 7, **characterized in that**, in case that said patient information and said photographing information, added by said adding means, include specific information, a memory means, for storing said image information to which said patient information and said photographing information including said specific information are added, is provided.

9. The image distributing apparatus, recited in claim 7 or claim 8, **characterized in that** said determining means determines a plurality of destination terminals by using said requesting information, and then, said distributing means distributes said image information to said plurality of destination terminals determined by said determining means.

10. The image distributing apparatus, recited in claim 9, **characterized in that** said determining means determines an image viewer equipped in a medical room, an image viewer equipped in an image inspection room, an image viewer equipped in a surgical operation room or an image server for controlling said image information as one of destination terminals by using said requesting information.

11. The image distributing apparatus, recited in anyone of claims 7-10, **characterized in that** said requesting information include information of a requested medical section or a requested doctor who requests a photographing operation of said patient.

12. The image distributing apparatus, recited in claim 7, **characterized in that** said photographing apparatus is a Computer Tomographic Scanner, a Magnetic Resonance image-forming apparatus or an X-ray image-forming apparatus (including Computed Radiography).

13. The image distributing apparatus, recited in claim 12, **characterized in that** said patient information and said photographing information are transferred to said image-forming apparatus from a terminal device equipped at a destination terminal, and are added to said image information.
